# EUROPÄISCHE PATENTSCHRIFT

(11) **EP 2 421 583 B1**
(45) Veröffentlichungstag und Bekanntmachung des Hinweises auf die Patenterteilung: **27.05.2020**
(21) Anmeldenummer: 10715693.7
(22) Anmeldetag: 14.04.2010
(51) Int. Cl.: A61M 1/36, A61M 1/34, A61M 5/165, A61M 1/00

(54) **EINRICHTUNG SOWIE EXTERNE FUNKTIONSEINRICHTUNG UND BEHANDLUNGSVORRICHTUNG ZUM BEHANDELN VON MEDIZINISCHEN FLUIDEN**
DEVICE, EXTERNAL FUNCTIONAL DEVICE AND TREATMENT DEVICE FOR TREATING MEDICAL FLUIDS
DISPOSITIF, DISPOSITIF FONCTIONNEL EXTERNE AINSI QU'APPAREIL DE TRAITEMENT POUR LE TRAITEMENT DE FLUIDES MÉDICAUX

(30) Priorität: 23.04.2009 DE 102009018664; 10.06.2009 DE 102009024467; 10.06.2009 US 185604 P
(43) Veröffentlichungstag der Anmeldung: 29.02.2012
(73) Patentinhaber: Fresenius Medical Care Deutschland GmbH, 61352 Bad Homburg v.d.H. (DE)
(72) Erfinder: LAUER, Martin, 66606 St. Wendel (DE)
(74) Vertreter: Bobbert & Partner Patentanwälte PartmbB
(86) Internationale Anmeldenummer: PCT/EP2010/002296
(87) Internationale Veröffentlichungsnummer: WO 2010/121742

(56) Entgegenhaltungen:
- WO-A2-01/37922
- DE-A1- 19 733 407
- US-A- 4 826 494
- US-A- 5 496 299

## Beschreibung

Die vorliegende Erfindung betrifft eine externe Funktionseinrichtung gemäß Anspruch 1 sowie eine Behandlungsvorrichtung gemäß Anspruch 14.

Verschiedene Behandlungsvorrichtungen, wie beispielsweise solche der Medizintechnik, weisen Einrichtungen auf, in welchen Fluide behandelt oder vorübergehend gespeichert werden sollen.

Die US 5,496,299 offenbart ein Absaugreservoir.

Die US 4,826,494 offenbart eine Einrichtung zur Wunddrainage.

Die WO 01/37922 A2 offenbart eine Wundenbehandlungsvorrichtung.

Die DE 197 33 407 A1 offenbart ein Filtrationsgerät.

Aufgabe der vorliegenden Erfindung ist, eine weitere zu diesem Zweck geeignete externe Funktionseinrichtung anzugeben.

Diese Aufgabe wird durch eine externe Funktionseinrichtung mit den Merkmalen des Anspruchs 1 gelöst.

Bei allen folgenden Ausführungen ist der Gebrauch des Ausdrucks "kann sein / haben" synonym zu "ist / hat vorzugsweise" zu verstehen.

Die erfindungsgemäße externe Funktionseinrichtung weist wenigstens eine Fluidaufnahmekammer, die zum Aufnehmen wenigstens eines ersten medizinischen Fluids geeignet und ausgelegt ist, auf. Sie weist ferner wenigstens eine hydrophobe Filtereinrichtung zum Zuführen eines zweiten gasförmigen Fluids in ein Inneres der Fluidaufnahmekammer auf.

Der Begriff "Fluidaufnahmekammer", wie er hierin verwendet wird, bezeichnet eine Kammer oder einen Behälter, welche/r ein Inneres oder einen Innenraum aufweist, der zum vollständigen oder teilweisen Befüllen mit Fluiden und Aufnehmen derselben geeignet und bestimmungsgemäß vorgesehen ist.

Ein "erstes Fluid" im Sinne der vorliegenden Erfindung schließt jede medizinische Flüssigkeit und/oder jedes medizinische Gas sowie beliebige Kombinationen ein, welche(s) zum Einbringen in eine erfindungsgemäße Aufnahmeeinrichtung angedacht bzw. vorgesehen sind. Vorzugsweise handelt es sich bei dem ersten Fluid um Blut.

Der Begriff "erstes Fluid" wird deshalb gleichbedeutend mit dem Begriff "medizinisches Fluid" verwendet.

Ein "zweites Fluid" ist ein gasförmiges Fluid, vorzugsweise Luft.

Der Begriff "Filtereinrichtung" oder "hydrophobe Filtereinrichtung", wie er hierin gleichbedeutend verwendet wird, bezeichnet eine Einrichtung, welche dazu ausgelegt und vorgesehen ist, eine Filterwirkung auf Fluide, welche durch die Fluidaufnahmekammer hindurchzuführen sind, auszuüben. Alternativ bezeichnet eine Filtereinrichtung i.S.d. Erfindung eine Membran ohne Filterwirkung.

Die Filterwirkung kann z. B. eine Reinigung der der Fluidaufnahmekammer zuzuführenden Fluide, insbesondere zum Beispiel das Zurückhalten von Feststoffen, Kleinstlebewesen, wie Viren oder Bakterien, und dergleichen umfassen.

Die Filtereinrichtung ist erfindungsgemäß derart angeordnet, dass ein Normalenvektor auf die Filteroberfläche der hydrophoben Filtereinrichtung nicht parallel zu einem Normalenvektor auf die Ebene des Fluidpegels bzw. des Fluidspiegels des im Gebrauch in der Fluidaufnahmekammer vorhandenen ersten Fluids verläuft. Die beiden Normalenvektoren liegen, anders ausgedrückt, nicht in einer Ebene.

Der Begriff "Normalenvektor", wie er hierin in Bezug auf die Filteroberfläche verwendet wird, bezeichnet einen Normalenvektor zu einem beliebigen Oberflächenabschnitt der Filtereinrichtung. Der Normalenvektor auf die Filteroberfläche kann eine Senkrechte zu einer Haupterstreckungsebene der Filtereinrichtung, besonders bevorzugt eine Senkrechte zu einer Oberfläche eines Hauptabschnitts der Filtereinrichtung, ganz besonders bevorzugt eine Senkrechte zu einem mittleren Abschnitt der Filtereinrichtung oder eine Senkrechte zu einem filternden Abschnitt der Filtereinrichtung oder der nichtfilternden Membran sein. Ein solcher Normalenvektor auf die Filteroberfläche kann ein senkrechtes Lot oder eine Senkrechte auf einen der zuvor genannten Abschnitte darstellen.

Der Begriff "Normalenvektor auf die Ebene des Fluidspiegels", wie er hierin verwendet wird, bezeichnet - in Anlehnung an die vorstehend angegebene Definition für den Normalenvektor auf die Filteroberfläche - einen Normalenvektor zu einem beliebigen Abschnitt bzw. Bereich der Ebene des Fluidspiegels.

Der Begriff "Fluidspiegel" bezieht sich erfindungsgemäß auf einen Fluidspiegel oder - pegel (diese beiden Begriffe werden nachfolgend synonym zueinander verwendet) eines in der Fluidaufnahmekammer vorhandenen ersten Fluids.

Der Fluidspiegel ist dabei auf ein Inneres der Fluidaufnahmekammer begrenzt. Er setzt sich nicht in Bereichen außerhalb der Fluidaufnahmekammer fort. Unter dem Fluidspiegel ist vorzugsweise nicht die gesamte Ebene durch den Fluidspiegel, sondern nur der von Wänden oder anderen Strukturen begrenzte Bereich dieser Ebene im Inneren der Fluidaufnahmekammer zu verstehen.

Der Fluidspiegel ist bevorzugt auf eine Kontaktfläche oder einen Kontaktabschnitt des ersten Fluids mit einem zweiten Fluid, welches ebenfalls im Inneren der Fluidaufnahmekammer vorliegt, beschränkt. Er hat bevorzugterweise keine weitere Ausdehnung hierüber hinaus. Der Fluidspiegel liegt somit bevorzugt allein innerhalb der Fluidaufnahmekammer vor.

Bei Schwapp- und/oder Strömungsbewegungen, beispielsweise Rotations- und/oder Wellenbewegungen der in der Fluidaufnahmekammer befindlichen Fluide kann es schwierig sein, einen Fluidspiegel oder eine Senkrechte hierauf zu bestimmen. Daher wird unter einem Fluidspiegel erfindungsgemäß bevorzugt ein unter Berücksichtigung aller Schwapp- und/oder Strömungsbewegungen der in der Fluidaufnahmekammer befindlichen Fluide gemittelter Durchschnittspegel oder -spiegel verstanden.

Der Fluidspiegel kann vorzugsweise in einem Strömungs-Ruhezustand des Fluids bestimmt werden. Es ist unerheblich, ob ein solcher Ruhezustand im Gebrauch der Einrichtung erreicht wird. Es genügt für die vorliegenden Zwecke, einen solchen anzunehmen oder zu approximieren.

Vorteilhafte Weiterentwicklungen der vorliegenden Erfindung sind jeweils Gegenstand der Unteransprüche.

In einer bevorzugten Ausführungsform steht der Normalenvektor auf die Filteroberfläche im Wesentlichen senkrecht zum Normalenvektor auf die Ebene des Fluidspiegels.

Der Begriff "im Wesentlichen senkrecht", wie er hierin verwendet wird, umfasst Abweichungen von der Rechtwinkeligkeit, die z. B. daher rühren, dass die externe Funktionseinrichtung - zum Beispiel eine Blutbehandlungskassette - welche die erfindungsgemäße Einrichtung aufweist, im Gebrauch eine geringe Neigung, z.B. bis zu +/- 15°, aufweist, während bei einer solchen Neigung der Filtereinrichtung der Fluid- oder Flüssigkeitspegel in der externen Funktionseinrichtung trotzdem horizontal bleibt.

In einer weiteren bevorzugten Ausführungsform der Erfindung hat ein Normalenvektor auf die Filteroberfläche keinen Schnittpunkt mit einem Fluidspiegel - wie oben definiert - des in der Fluidaufnahmekammer vorhandenen ersten Fluids.

Erfindungsgemäß weist die Einrichtung wenigstens eine Fluidzuführungskammer zum Zuführen des zweiten Fluids auf.

Der Begriff "Fluidzuführungskammer", wie er hierin verwendet wird, bezeichnet eine Kammer oder einen Behälter, welche/r ein Inneres oder einen Innenraum aufweist, der zum Aufnehmen eines zweiten Fluids und Zuführen desselben in die Fluidaufnahmekammer geeignet und bestimmt ist.

Die Fluidzuführungskammer kann in Form einer Spritzgusskammer hergestellt sein. Die Fluidzuführungskammer kann eine Einmal-Fluidzuführungskammer sein.

Die Fluidzuführungskammer ist in wenigstens einem Abschnitt derselben mit der Fluidaufnahmekammer verbunden.

Sie kann stoffschlüssig mit der Fluidaufnahmekammer verbunden oder in diese integriert sein.

Die Fluidzuführungskammer kann beispielsweise während der Herstellung der Fluidaufnahmekammer ausgestaltet werden.

Die Fluidzuführungskammer kann durch wenigstens eine Abgrenzung oder eine Teilwand von der Fluidaufnahmekammer abgetrennt sein.

In einer weiteren bevorzugten Ausführungsform der vorliegenden Erfindung ist die Filtereinrichtung in einem Inneren der Fluidzuführungskammer angeordnet.

Die Fluidzuführungskammer und die Fluidaufnahmekammer stehen erfindungsgemäß lediglich über die Filtereinrichtung miteinander in Fluidkommunikation. Bevorzugt wird dabei das zweite Fluid aus der Fluidzuführungskammer durch die Filtereinrichtung in die Fluidaufnahmekammer eingebracht bzw. dieser zugeführt.

Ganz besonders bevorzugt ist die Filtereinrichtung derart ausgestaltet und vorgesehen, dass ein in der Fluidaufnahmekammer befindliches erstes Fluid nicht durch die Filtereinrichtung in die Fluidzuführungskammer gelangen kann.

Das der Fluidaufiiahmekammer zuzuführende zweite Fluid kann hierzu die Filtereinrichtung durchtreten oder durchströmen. Vorzugsweise wird es in der bzw. durch die Filtereinrichtung vorteilhaft von unerwünschten Feststoffen, unerwünschten Fluiden, Bakterien und dergleichen gereinigt. Auf diese Weise kann vorteilhaft verhindert werden, dass unerwünschte Stoffe aus einem Äußeren der Einrichtung in die Fluidaufnahmekammer eingebracht oder verschleppt werden. Auch eine Ausbreitung derselben ausgehend von der Fluidaufnahmekammer kann vorteilhaft vermieden werden.

Das zweite Fluid wird über einen Fluidkonnektor, welcher mit der Fluidzuführungskammer verbunden ist, in die Fluidzuführungskammer eingebracht, eingeströmt, eingeleitet oder dergleichen.

Der Fluidkonnektor kann ein hülsenförmiges Gebilde sein. Er kann aus einem Stück mit einer Wandung bzw. Seitenwand der Fluidzuführungskammer hergestellt sein. Er kann auf bzw. an einer Außenseite der Wandung oder Seitenwand der Fluidzuführungskammer vorgesehen sein. Er kann innerhalb eines Bereichs der in der Fluidzuführungskammer angeordneten Filtereinrichtung vorgesehen sein. Er kann direkt mit der Filtereinrichtung verbunden bzw. an diese angeschlossen sein.

Der Fluidkonnektor kann über eine oder mehrere Verbindungsbohrungen mit einer Innenseite der Wandung oder Seitenwand der Fluidzuführungskammer verbunden sein. In jeder erfindungsgemäßen Ausführungsform kann die Fluidaufnahmekammer beispielsweise in Form einer Spritzgusskammer hergestellt sein.

Die Fluidaufnahmekammer kann eine Einmal-Fluidaufnahmekammer sein.

Die Fluidaufnahmekammer kann eine Fluidverbindung zu einem Äußeren der Kammer aufweisen. Sie kann zwei oder mehr Fluidverbindungen zum Äußeren der Kammer aufweisen.

Erfindungsgemäß weist die Filtereinrichtung wenigstens eine Filtermembran auf.

Eine "Filtermembran" ist vorzugsweise dazu ausgelegt und vorgesehen unerwünschte Fremdstoffe und dergleichen aus dem der Fluidaufnahmekammer zuzuführenden zweiten Fluid herauszufiltern. Eine Filterfunktion ist jedoch erfindungsgemäß keine zwingend erforderliche Eigenschaft der Membran. Die Filtermembran weist übliche, für eine Membran bekannte Eigenschaften auf.

Die Fluidaufnahmekammer ist mittels der Filtermembran bevorzugt vom Fluidkonnektor der Fluidzuführungskammer und/oder von einem Äußeren der Einrichtung entkoppelt. Die Filtermembran kann eine beliebige geeignete Form aufweisen. Sie kann beispielsweise rund, eckig, insbesondere rechteckig, elliptisch und dergleichen ausgestaltet sein.

Die Filtermembran kann aus einem Filtermembranband ausgeschnitten und/oder gegebenenfalls auf eine bestimmte Form zugeschnitten werden oder sein.

Die Filtermembran kann eine Einmal-Filtermembran sein.

Die Filtermembran kann eine hydrophobe Membran sein. Die Filtermembran kann an wenigstens einer Seite hydrophob sein.

Die Filtermembran kann aus zwei Schichten bestehen, nämlich erstens der eigentlichen Membran, die zumeist aus einem schwer schweißbaren und schwer klebbaren Werkstoff besteht, wie z. B. PTFE (Polytetrafluorethylen), und zweitens einer Schicht, die eine Drainage- und/oder Stützfunktion hat und zumeist aus Gewebe und/oder Vlies besteht, das schweißbar und/oder klebbar ist. Die Filtermembran kann alternativ neben den zuvor genannten Schichten weitere Schichten oder Bestandteile aufweisen.

Die Filtermembran kann eine Sterilmembran sein.

Die Filtermembran kann aus einem nicht oder nur schwierig verklebbaren und/oder einem nicht oder nur schwierig verschweißbaren Werkstoff bestehen oder einen solchen aufweisen.

Die Filtermembran kann stoffschlüssig (z. B. durch Schweißen und/oder Kleben) und/oder kraftschlüssig (z. B. durch Verpressen mit einem O-Ring) und/oder formschlüssig mit der Fluidzuführungskammer verbunden oder integriert sein.

Die Filtermembran kann während der Verwendung der Einrichtung, wie beispielsweise der Dauer einer Behandlung, wechselnden Drücken aus Richtung der Fluidaufnahmekammer (ausströmendes Gas, anstehende Flüssigkeit) und/oder aus Richtung einer Behandlungsvorrichtung (einströmendes Gas, anstehende Störfallflüssigkeit und/oder aus Kondensation entstehende unerwünschte Flüssigkeit) ausgesetzt sein. Zum Schutz der Filtermembran im flächigen Nutzbereich und/oder an den Stellen ihrer Abdichtung (in der Regel Verschweißung) z. B. an Wandungen der Fluidzuführungskammer vor Verformung durch diese Druckdifferenzen, welche zu einem Strukturschaden oder einem Riss in der Membran führen könnte, kann es von Vorteil sein, die Filtermembran mechanisch abzustützen.

Zum Erzielen einer solchen mechanischen Abstützung kann eine Stützstruktur eingesetzt werden. Diese ist derart ausgestaltet, dass die Stützflächen die Filtermembran nicht unzulässig gegen den gewünschten Fluiddurchtritt durch die Filtermembran, z. B. einen Nutzgasdurchtritt, abdichten.

Die Filtereinrichtung kann symmetrisch oder asymmetrisch aufgebaut sein.

Erfindungsgemäß weist die Filtereinrichtung auf bzw. an wenigstens einer Seite oder beiden Seiten eine Stützstruktur auf.

Eine erste und eine zweite Stützstruktur können auf beiden Seiten der Filtermembran vorgesehen sein. Sie können getrennt von dieser vorliegen. Eine dritte Stützstruktur kann auf der Membranschicht angeordnet sein. Sie kann vorzugsweise als Vlies, Gewebe oder dergleichen ausgestaltet sein.

Die erste Stützstruktur ist vorzugsweise im Inneren des Gehäuses des Fluidkonnektors vorgesehen. Sie kann eine Drainagestruktur aufweisen. Die erste Stützstruktur kann eine Drainagewirkung vermitteln.

Die erste Stützstruktur kann kraft- und/oder form- und/oder stoffschlüssig mit der Fluidzuführungskammer verbunden sein. Bevorzugt ist die erste Stützstruktur in einem äußeren umlaufenden Bereich kraft- und/oder form- und/oder stoffschlüssig mit der Fluidzuführungskammer verbunden.

Da die Filtermembran in der Regel aus einem nicht oder schlecht verklebbaren und/oder einem nicht oder schlecht verschweißbaren Werkstoff besteht, kann die dem Inneren der Fluidzuführungskammer zugewandte Stützstruktur in wenigstens einem äußeren Bereich, bevorzugt einem äußeren, umlaufenden Bereich, derselben mit der Fluidzuführungskammer verbunden sein.

Bevorzugterweise ist die erste Stützstruktur stoffschlüssig mit der Fluidzuführungskammer verbunden, beispielsweise verschweißt, z. B. mittels Thermoschweißen. Die erste Stützstruktur kann thermisch mit der Fluidzuführungskammer bzw. einem Verbindungsbereich derselben verschweißt sein. Zu diesem Zweck ist die erste Stützstruktur - bevorzugt in einem äußeren Bereich derselben - aus einem höher schmelzenden Werkstoff als der Verbindungsbereich der Fluidzuführungskammer hergestellt.

Beim Verschweißen der ersten Stützstruktur mit der Fluidzuführungskammer bzw. einem Verbindungsbereich derselben kann der durch Erwärmen verflüssigte Werkstoff der Fluidzuführungskammer in eine poröse Struktur der Stützstruktur eindringen. Der flüssige Werkstoff kann bis zur Filtermembran eindringen. Auf diese Weise kann vorteilhaft eine unlösbare Verbindung zwischen der Fluidzuführungskammer, der ersten Stützstruktur und der Filtermembran gebildet werden. Gleichzeitig kann die Filtermembran besonders seitlich vorteilhaft abgedichtet werden.

Die zweite Stützstruktur ist vorzugsweise ein dünnwandiges Spritzgussteil. Sie weist bevorzugt auf der der Membranschicht zugewandten Seite oder beiden eine Drainagestruktur auf.

Die erste und/oder zweite Stützstruktur können mittels Spritzguss hergestellt sein.

Beispielsweise kann die erste Stützstruktur in eine spritzgusstechnisch erzeugte Wandung der Fluidzuführungskammer integriert sein.

In einer weiteren bevorzugten Ausführungsform weist die Filtermembran an oder auf der dem Inneren der Fluidzuführungskammer abgewandten Seite der Filtermembran eine zweite Stützstruktur auf.

Die zweite Stützstruktur kann im Wesentlichen parallel zu der Filtermembran angeordnet sein.

Die zweite Stützstruktur kann kraftfrei an der Filtermembran anliegend und/oder mit geringem Spiel an der dem Inneren der Fluidzuführungskammer abgewandten Seite der Filtermembran vorgesehen sein.

Die zweite Stützstruktur kann aus dem gleichen Werkstoff wie die Fluidzuführungskammer hergestellt sein.

Die zweite Stützstruktur kann ein separates Element bilden bzw. als solches hergestellt sein. Beispielsweise kann die zweite Stützstruktur als dünnwandiges Spritzgussbauteil gefertigt sein.

In einer weiter bevorzugten Ausführungsform überdeckt die zweite Stützstruktur die Filtermembran im Wesentlichen vollständig.

Die vom Inneren der Fluidzuführungskammer abgewandte zweite Stützstruktur kann in ihrer Fläche und/oder Berandung nach Außen durch eine Ringzone oder einen Ringbereich ohne Drainagewirkung abgeschlossen sein.

Die Begriffe "Ringzone" oder "Ringbereich" bezeichnen einen äußeren Bereich oder äußeren Rand bzw. Außenrand der zweiten Stützstruktur. Der Wortteil "Ring-" soll die Erfindung dabei jedoch nicht auf eine kreisförmige Ausgestaltung der Zone oder des Bereichs einschränken. Vielmehr soll er einen umlaufenden Bereich oder eine umlaufende Zone beschreiben, welche jedoch auch in jeder beliebigen anderen geeigneten Form ausgestaltet sein kann, beispielsweise in Form eines Rechtecks, einer Ellipse und dergleichen.

Eine Außenberandung dieser Ringzone oder dieses Ringbereichs kann im Wesentlichen den Außenabmessungen der Filtermembran entsprechen.

Eine Innenberandung dieser Ringzone oder dieses Ringbereichs kann im Wesentlichen dem nach der Befestigung der Filtermembran an der Ringzone oder dem Ringbereich verbleibenden filterwirksamen Filtermembranareal entsprechen.

Die Filtermembran kann gasdicht mit der zweiten Stützstruktur verbunden sein. Sie kann mit einem Wandungsmaterial der Ringzone oder des Ringbereichs verbunden sein. Beispielsweise kann die Filtermembran in einem äußeren, bevorzugt umlaufenden, Bereich derselben mit der zweiten Stützstruktur bzw. einem äußeren Ringbereich derselben verbunden sein.

Die Filtermembran kann stoffschlüssig mit dem Wandungsmaterial verbunden sein. Sie kann beispielsweise mit dem Wandungsmaterial verklebt oder verschweißt sein.

Die Filtermembran kann entsprechend sowohl mit der ersten Stützstruktur als auch mit der zweiten Stützstruktur verbunden oder integriert sein.

Die zweite Stützstruktur kann ein dünnwandiges Spritzgussteil bzw. -element sein, welches mittels Verschweißen und/oder Verkleben mit der Filtermembran und/oder dem Gehäuse der Filtereinrichtung verbindbar ist.

Ein wenigstens drei Stützstrukturen aufweisendes Konstrukt der erfindungsgemäßen Einrichtung kann beispielhaft wie folgt gebildet werden:
Auf die Filtermembran wird die dritte Stützschicht aufgebracht. Die dritte Stützschicht, z. B. ein schweißbares Vlies, wird bei der Montage auf der Seite des Vlieses auf die erste Stützstruktur gelegt. Die dritte Stützschicht wird am Umfang der Filtermembranschicht, d. h. vorzugsweise in dem Bereich, welcher außerhalb der ersten Stützstruktur liegt, umlaufend dicht mit der ersten Stützstruktur verschweißt und/oder verklebt. Auf diese Weise kann vorteilhaft eine Dichtfunktion zwischen der dritten Stützstruktur und der ersten Stützstruktur erreicht werden.

Die zweite Stützstruktur, z.B. ein dünnwandiges Spritzgussteil, wird wie ein Deckel mit ihrer Stützstruktur auf die an der ersten Stützstruktur angeschweißte Filtermembran gelegt. Sie wird in einer äußeren Ringzone außerhalb der Filtermembranschicht mit dem Gehäuse (Spritzgussgehäuse) des Fluidkonnektors verschweißt und/oder verklebt.

Die zweite Stützstruktur stellt vorteilhaft eine Haltefunktion der Filtermembran bereit.

Die erste und/oder zweite Stützstruktur(en) können derart ausgestaltet sein, dass nach Anordnen der Stützstruktur(en) noch unabgestützt frei zugängliche Flächenanteile der Filtermembran eine jeweils hinreichend kleine Ausdehnung bis zu den benachbarten mechanischen Abstützungen oder Stützstrukturen aufweisen.

Der maximal zulässige Fluiddruck des zweiten Fluids auf die freien Filtermembranflächen erzeugt damit bevorzugt keine unzulässig hohe Spannung (beispielsweise bedingt durch Ausbeulung der Filtermembran) mehr.

Beispielsweise können einzelne oder alle Stützstrukturen, wie beispielsweise Drainagestrukturen, eine Breite von etwa 0,5 bis 2 mm aufweisen.

Der außenseitig angeordnete Fluidkonnektor kann über Bohrungen, Aussparungen oder Öffnungen mit der außenseitigen Drainage- oder Stützstruktur für die Filtermembran, d. h. der ersten Stützstruktur, in Verbindung stehen.

Die zwischen der ersten und der zweiten Stützstruktur angeordnete Filtermembran kann im Wesentlichen bis auf ein gebrauchsbestimmt erforderliches Maß mechanisch belastbar sein.

Die Ebene, in welcher eine derartige Membranverbindung, d. h. eine Verbindung zwischen der Filtermembran und den beiden Stützstrukturen, angeordnet ist, kann im Wesentlichen der Ebene entsprechen, in welcher erhabene Bereiche der Drainagestruktur angeordnet sind.

Je nach Verbindungsverfahren und/oder Dicke der Filtermembran kann ein Höhenversatz zwischen erhabenen Bereichen der Drainagestruktur und der außenseitigen Ebene der Filtermembran sinnvoll sein. Ein solcher Höhenversatz kann dazu dienen, die außenseitige Ebene der Filtermembran kraftfrei und/oder mit leichtem Spiel auf den erhabenen Drainagestrukturen aufliegen zu lassen.

Die erhabenen Drainagestrukturen können so klein wie möglich angeordnet sein.

Das zweite Fluid kann vorzugsweise nur beschränkt durch die Filtermembranbereiche strömen, welche durch Anlage an die erhabenen Drainagestrukturen in Kontakt und/oder in Verpressung mit diesen gelangen.

Die Größe und/oder Anzahl der Verbindungsbohrungen zum Fluidkonnektor und/oder die Anordnung, Anzahl, Breite und/oder Tiefe der vertieften Drainagestrukturen können derart sein, dass ein möglicher durch diese Strömungswege verursachter Druckabfall des zweiten Fluids ein vernachlässigbarer oder hinnehmbarer Bruchteil des Gesamtdruckabfalls beim Durchströmen der Filtereinrichtung ist.

Die Breite der vertieften Drainagestrukturen und/oder die Durchmesser der Verbindungsbohrungen zum Fluidkonnektor können hinreichend klein ausgelegt sein derart, dass die bei maximal möglichen Druckdifferenzen auf die Filtermembran wirkenden Zugkräfte (welche beispielsweise zu einer Ausbeulung in die vertieften Strukturen führen) deutlich unterhalb der zulässigen Zugkräfte bevorzugt sowohl innerhalb der Filtermembran als auch an den in der Regel empfindlicheren Zonen am Übergang zur Ringbefestigung (zum Beispiel Schweißnaht) liegen.

In einer weiteren bevorzugten Ausführungsform weisen die erste und die zweite Stützstruktur eine zu einer Hauptebene der Membran spiegelbildlich identische oder im Wesentlichen identische Drainagestruktur auf.

Bevorzugt liegen sich die vertieften und/oder die erhabenen Drainagestrukturen deckungsgleich oder im Wesentlichen deckungsgleich gegenüber.

Bevorzugt sind die vertieften Drainagestrukturen auf der einen Filtermembranseite schmäler bzw. die erhabenen Drainagestrukturen auf dieser Filtermembranseite breiter ausgeführt als die Drainagestrukturen auf der anderen Filtermembranseite. Dies kann eine größere laterale Einbautoleranz erlauben. Die Teilungsabstände und/oder Anordnungen der Strukturen können jedoch auf beiden Seiten bevorzugt identisch ausgeführt sein.

Auf diese Weise kann sich bei Ausnutzung der lateralen Einbautoleranzen ein sehr konstantes Eigenschaftsprofil hinsichtlich Merkmalen wie Fluiddurchtrittswiderstand und Grad der mechanischen Abstützung ergeben.

Die Gesamtstärke der Struktur der zweiten Stützstruktur kann sich aus der Tiefe der Drainagestruktur und/oder der minimal möglichen Wandstärke des Materials der zweiten Stützstruktur ergeben oder die Summe hieraus sein. Dadurch kann vorteilhafterweise die zweite Stützstruktur wenig Bauraum benötigen und/oder günstiger hergestellt werden.

Ein weiterer Vorteil kann darin bestehen, dass an die zweite Stützstruktur keine besonderen Anforderungen an Präzision und Steifigkeit bestehen. Zudem kann es vorteilhaft möglich sein, die zweite Stützstruktur an der Fluidzuführungskammer einzig nach Kostengesichtspunkten und/oder möglichst geringem Aufwand zu befestigen.

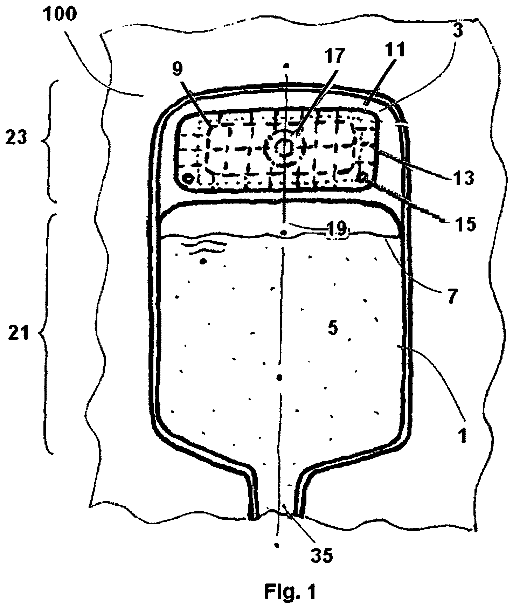

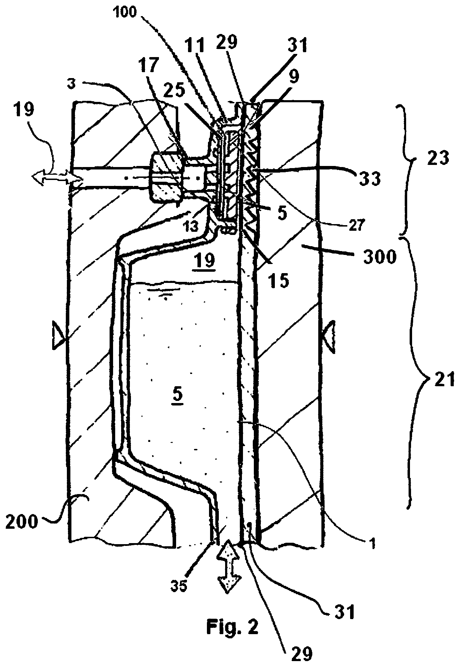

Wie in den Fig. 1 und 2 gezeigt ist, kann die zweite Stützstruktur beispielsweise mittels einer Steck- oder Nietbefestigung oder dem Prinzip der Bolzenhalterung mit der Fluidzuführungskammer verbunden sein. Ebenso kann die zweite Stützstruktur mit der Fluidzuführungskammer mittels punktförmigem Anschweißen und/oder Einklipsen in geeignete geometrische Ausgestaltungen einer Seitenwand oder Wandung der Fluidzuführungskammer verbunden sein.

Die Drainagestrukturen der zweiten Stützstruktur können sich in einer erfindungsgemäßen Ausführungsform von den außenseitigen Drainagestrukturen u. a. oder allein darin unterscheiden, dass erstere nach außen hin nicht an den Membrangrenzen enden, sondern radial nach außen bis an die Bauteilberandung durchgeführt sind. Damit können ein- und/oder ausströmende Fluide ungehindert in den verbleibenden Ringraum zwischen dem zweiten Stützgitter und einer oberen Berandung bzw. einem oberen Rand der Fluidzuführungskammer dringen. Die Fluide können vorteilhaft großlumig mit der Fluidaufnahmekammer in Verbindung stehen.

In einer weiter bevorzugten Ausführungsform der vorliegenden Erfindung weist die Fluidaufnahmekammer eine erste Bauhöhe auf, und die Fluidzuführungskammer weist eine zweite Bauhöhe auf, welche von der ersten Bauhöhe verschieden ist.

Auch oder zusätzlich dazu kann die Fluidzuführungskammer während der Verwendung der Einrichtung oberhalb der Fluidaufnahmekammer ("oben") angeordnet sein. "Oberhalb" kann sich auf ein als durch den Erdmittelpunkt verlaufend gedachtes Bezugssystem beziehen.

In einer derartigen Anordnung der Fluidaufnahmekammer und der Fluidzuführungskammer ist die Einrichtung bevorzugt in gestufter Tiefe ausgeführt. Die während der Verwendung der Einrichtung unterhalb der Fluidzuführungskammer ("unten") angeordnete tiefe Fluidaufnahmekammer kann im Gebrauch als Reservoir und/oder Behandlungsraum für die darin befindlichen Fluide genutzt werden.

Die erfindungsgemäße Einrichtung kann in Kassettenbauform ausgestaltet sein. Sie kann beispielsweise Teil einer externen Funktionseinrichtung sein. Die Einrichtung kann beispielsweise stoffschlüssig in die externe Funktionseinrichtung integriert sein.

Die externe Funktionseinrichtung kann an wenigstens einer Seite mit einem Abdeckungselement versehen sein.

Ein "Abdeckungselement" kann beispielsweise eine Membran, eine Folie und dergleichen sein. Beispielhafte Ausführungen für geeignete Abdeckungselemente sowie deren Ausgestaltung und Anordnung an der externen Funktionseinrichtung können beispielsweise der von der Anmelderin der vorliegenden Erfindung beim DPMA am 10. März 2009 eingereichten Anmeldung 10 2009 012 632 A1 mit dem Titel *"4bdichtungseinrichtung zum Abdichten eines Volumens einer medizinischen Behandlungsanordnung gegen ein weiteres Volumen sowie Anordnung und Verfahren*" entnommen werden, auf deren diesbezügliche Offenbarung hiermit vollinhaltlich Bezug genommen wird.

Besonders bevorzugt ist die Filtereinrichtung parallel oder im Wesentlichen parallel zum Abdeckungselement der externen Funktionseinrichtung angeordnet.

Die externe Funktionseinrichtung kann zum Behandeln des ersten Fluids geeignet und vorgesehen sein.

Eine solche Behandlung kann beispielsweise erfolgen, indem das Volumen und/oder der Druck des ersten Fluids in der Fluidaufnahmekammer durch Befüllen, Entleeren und/oder Druckbeaufschlagung variiert werden. Dazu kann beispielsweise das erste Fluid in der Fluidaufnahmekammer von einem Volumen des zweiten Fluids überlagert sein. Das zweite Fluid kann beispielsweise die zuvor genannten Funktionen über eine Konnektion zu einer Behandlungsvorrichtung, welche entsprechende Aktoren und/oder Steuer- oder Regeleinrichtungen aufweisen kann, auf die Flüssigkeit übertragen.

In einer besonders bevorzugten Ausführungsform der vorliegenden Erfindung ist das zweite Fluid ein Gas. Weiter bevorzugt kann das erste Fluid eine Flüssigkeit, wie beispielsweise Blut, sein.

Die erfindungsgemäße Einrichtung ist zum Einsatz in bzw. an bzw. mit einer Behandlungsvorrichtung, wie einer medizinischen Behandlungsvorrichtung, einer Vorrichtung aus der Labortechnik, einer Vorrichtung der Nahrungsmittel- und/oder Arzneimittelherstellung geeignet. Zum Einbringen bzw. Einleiten oder Einführen in die erfindungsgemäße Aufnahmeeinrichtung geeignete Fluide können daher sowohl medizinische Flüssigkeiten wie Blut, Substituat (z. B. Kochsalzlösung), Wirkstoffzubereitungen, wie Lösungen, Suspensionen, Emulsionen, Trägergase für Wirkstoffe, Reinigungsflüssigkeiten oder -gase, Desinfektionsflüssigkeiten oder -gase, Sterilisationsflüssigkeiten oder -gase, Getränkeflüssigkeiten und dergleichen einschließen.

Wenn die Filtermembran als Sterilmembran vorgesehen ist, kann die erfindungsgemäße Einrichtung insbesondere zur Sterilluftversorgung der Fluidaufnahmekammer eingesetzt werden.

Die erfindungsgemäße Aufgabe wird durch eine externe Funktionseinrichtung gemäß Anspruch 1 gelöst. Alle Vorteile der erfindungsgemäßen Aufnahmeeinrichtung lassen sich auch mit der erfindungsgemäßen externen Funktionseinrichtung erreichen.

Eine erfindungsgemäße externe Funktionseinrichtung weist eine erfindungsgemäße Einrichtung auf.

Die erfindungsgemäße externe Funktionseinrichtung kann zur Verwendung in einem Behandlungsverfahren angedacht sein. Behandlungsverfahren im Sinne der vorliegenden Erfindung schließen medizinische oder medizintechnische Behandlungsverfahren, Behandlungsverfahren der Labortechnik, der Nahrungs- oder Arzneimittelherstellung und dergleichen ein.

Eine solche externe Funktionseinrichtung kann eine Einmalkomponente oder ein Einmalartikel sein, welcher beispielsweise aus einem Kunststoffmaterial gefertigt ist.

Die externe Funktionseinrichtung kann mittels eines Spritzgussverfahrens hergestellt sein.

Die externe Funktionseinrichtung kann nach oben hin offen sein.

Die externe Funktionseinrichtung kann über Flüssigkeits- und/oder Gasanschlüsse, über halboffene Kanäle und/oder Kammern und/oder über Strukturen zur Ankopplung an Aktoren und/oder Sensoren verfügen. Derartige Aktoren und/oder Sensoren können zur Durchführung von bevorzugt nicht-invasiven und/oder steril entkoppelten Funktionen an den Flüssigkeiten in der Kassette dienen. Eine oder mehrere Abdeckungselemente, wie beispielsweise Membranen, insbesonders preisgünstige Folien, können für den Abschluss und/oder die Abdichtung der Kanäle und Kammern sorgen.

In einer weiter bevorzugten Ausführungsform ist die Filtereinrichtung der Einrichtung parallel zu einem Abdeckungselement der Einrichtung zum Verschließen eines Inneren der Fluidaufnahmekammer gegenüber einem Äußeren angeordnet.

In einer bevorzugten Ausführungsform ist die erfindungsgemäße externe Funktionseinrichtung als Blutkassette ausgestaltet.

Eine derartige Blutkassette ist beispielsweise in der von der Anmelderin der vorliegenden Erfindung in der beim DPMA am 23. April 2009 eingereichten Anmeldung 10 2009 018 664 A1 mit dem Titel *"Externe Funktionseinrichtung, Blutbehandlungsvorrichtung zum Aufnehmen einer erfindungsgemäßen externen Funktionseinrichtung, sowie Verfahren*" sowie der am 10. Juni 2009 mit gleichem Titel eingereichten Patentanmeldung 10 2009 024 468 A1 beschrieben, auf deren diesbezügliche Offenbarungen hiermit vollinhaltlich Bezug genommen wird.

Die externe Funktionseinrichtung kann zur Verwendung in oder an einer Behandlungsvorrichtung vorgesehen sein.

Die erfindungsgemäße Aufgabe wird daher gleichermaßen durch eine erfindungsgemäße Behandlungsvorrichtung gemäß Anspruch 14 gelöst. Alle Vorteile der erfindungsgemäßen Einrichtung lassen sich auch mit der erfindungsgemäßen Behandlungsvorrichtung erreichen.

Die erfindungsgemäße Behandlungsvorrichtung kann eine erfindungsgemäße Einrichtung und/oder eine erfindungsgemäße externe Funktionseinrichtung aufweisen.

Die Behandlungsvorrichtung kann in einem Behandlungsverfahren, wie es vorstehend angegeben ist, eingesetzt werden.

Beispielsweise kann die Behandlungsvorrichtung eine Blutbehandlungsvorrichtung, wie eine Dialysiervorrichtung, zum Durchführen einer Dialysebehandlung, wie einer Hämodialyse, Hämofiltration, Hämodiafiltration und dergleichen, sein.

Die erfindungsgemäße Einrichtung kann in vorteilhafter Weise zur Sterilluftversorgung einer Fluidaufnahmekammer eingesetzt werden.

Die Behandlungsvorrichtung kann wenigstens eine Aufnahmeeinrichtung zum Aufnehmen wenigstens einer Blutbehandlungskassette geeignet und vorgesehen sein, die wenigstens eine erfindungsgemäße Einrichtung aufweist.

Anders als bei herkömmlichen Anordnungen zur Sterilluftversorgung von externen Funktionseinrichtungen, bei welchen die hydrophoben Sterilmembranen am höchsten Punkt der Fluidaufnahmekammer und im Wesentlichen parallel zur freien Fluidoberfläche angeordnet sind, kann die erfindungsgemäß vorgesehene Filtermembran bevorzugt geodätisch oberhalb der normal maximal mit Fluiden gefüllten Fluidaufnahmekammer angeordnet sein. Im störungsfreien Betrieb kann die Fluidzuführungskammer daher vorteilhaft nur mit dem zweiten Fluid, zum Beispiel Gas, und mit geringen Mengen des ersten Fluids, z. B. einer Flüssigkeit wie Blut, in Kontakt kommen.

Da die erfindungsgemäß eingesetzten Filtermembranen als empfindliche und schwer überprüfbare Elemente in der Regel mit jedem Behandlungseinsatz ausgewechselt werden sollten, kann die Filtermembran vorteilhaft Bestandteil der Einmal-Funktionseinrichtung sein. Derartige Filtermembranen können ferner weiter vorteilhaft zugleich gemeinsam mit der Einmal-Funktionseinrichtung sterilisiert werden und/oder das Einmalteilsystem bei Lagerung und/oder während der Konnektion mit der Behandlungsvorrichtung steril verschlossen halten.

Hydrophobe Filtermembranen können ferner vorteilhaft dafür sorgen, dass bei Fehlsteuerungen die in der Fluidaufnahme-kammer vorhandenen, zu behandelnden Fluide nicht in die Behandlungsvorrichtung gelangen können.

Die erfindungsgemäße Einrichtung kann vorteilhaft die, in für Behandlungsvorrichtungen, insbesondere Blutbehandlungsvorrichtungen, oftmals vorgesehenen Einmal-Funktionseinrichtungen, vorhandenen räumlichen und/oder funktionalen Anordnungen zur Unterbringung, zur Verpressung, zum Toleranzausgleich, zur Kraftbegrenzung, zur Halterung, zur Ausrichtung und/oder zur Handhabung parallel zu anderen Funktionseinheiten der Gesamtanordnung nutzen.

Aus den unterschiedlichen geometrischen Umgebungsbedingungen herkömmlicher Anordnungen kann sich mit der Anordnung der Fluidzuführungskammer und der Fluidaufnahmekammer der erfindungsgemäßen Einrichtung durch räumliche Enge im Umfeld der Filtermembran vorteilhaft ein flächenspezifisch höherer Gasdruckverlust an den Drainage- oder Stützstrukturen ergeben. Auf diese Weise kann es vorteilhaft möglich sein, bei gleichem Druckverlust, preisgünstigere und kleiner zu bauende Filtermembranen einzusetzen, was aufgrund der Rechteckform häufig zugleich den Bauraum effektiver zu nutzen vermag.

Die Gaskonnektion zwischen Behandlungsvorrichtung und externer Funktionseinrichtung kann daneben vorteilhaft in im Wesentlichen gleicher Bewegungs- und Verpressungsrichtung wie der Einbau und die Verpressung der gesamten externen Funktionseinrichtung zwischen einer türseitigen Anpressstruktur der Behandlungsvorrichtung und einer korpusseitigen Anpressstruktur der Behandlungsvorrichtung erfolgen. Damit können durch Bewegung und/oder Kraft der Verpressungseinrichtungen oder Anpressstrukturen der Behandlungsvorrichtung ökonomisch beide genannten Funktionen parallel genutzt werden. Ein separater Handlingsvorgang zur Gaskonnektion der externen Funktionseinrichtung kann vorteilhaft entfallen. Durch starre und gleich gerichtete Parallelität der Verpressungs- und der Konnektionsvorgänge können vorteilhaft ferner enge Toleranzanforderungen zwischen der externen Funktionseinrichtung und der Behandlungsvorrichtung und zwischen einzelnen Komponenten der Behandlungsvorrichtung entfallen, welche nötig wären, wenn die Gaskonnektion über eine separate Bewegungs- und Verpressungseinrichtung in anderer räumlicher Richtung als die allgemeine Verpressung der externen Funktionseinrichtung realisiert würde.

Die erfindungsgemäße vorgesehene Gaskonnektoranordnung kann in vorteilhafter Weise zugleich platzsparend, preiswert, funktionssicher, robust sowie anwenderfreundlich ausgelegt sein.

Da mit der vorliegenden Erfindung die Anzahl an Funktionsgruppen reduziert werden kann, kann es auf vorteilhafte Weise möglich sein, Kosten u. a. bei Herstellung, Lagerung, Logistik und Montage einzusparen. Ferner kann mit der vorliegenden Erfindung eine Reduktion von Genauigkeitsansprüchen zwischen den einzelnen Funktionsgruppen erreicht werden, was weiter vorteilhaft zur Kostensenkung beitragen kann.

Die Handhabung der Einmal-Funktionseinrichtungen beim Auf- und Abrüsten derselben kann mit der vorliegenden Einrichtung vorteilhaft erleichtert und sicherer werden.

Durch den Wegfall hoher Steifigkeits-, Präzisions-, Material- und/oder Herstellprozessanforderungen bei der Herstellung der Komponenten der externen Funktionseinrichtung wie Fluidkonnektor, Stützstrukturen, Drainagestrukturen, Membranen, Filtermembranen und Membranbefestigungen und/oder -abdichtungen können sich in vorteilhafter Weise deutliche Senkungen bei den Herstellkosten der externen Funktionseinrichtungen bei gleichzeitiger Steigerung der Zuverlässigkeit in der Funktion ergeben.

Insbesondere die konsequent kraftgeregelte und/oder spielfreie Ankopplung der Filtermembran an ihre beidseitigen Drainage- oder Stützstrukturen kann ein besonders wertvolles Merkmal der erfindungsgemäßen Anordnung darstellen. Die Filtermembran kann deutlich belastbarer und funktionssicherer sein, als in herkömmlichen Anordnungen.

Während aus Festigkeitsgründen der umgebenden Stützkonstruktion in herkömmlichen Systemen die Filtermembran in der Regel rund ausgeführt werden muss und mit geringer lateraler Toleranz in die Gehäuseumgebung eingepasst werden muss, kann die Filtermembran in der erfindungsgemäßen Einrichtung verschnittfrei als Rechteck aus einem Filtermembranband hergestellt werden und unter großzügiger lateraler Toleranz auf die Wandung der Fluidzuführungskammer aufgeschweißt, aufgeklebt oder aufgepresst werden.

Im Gegensatz zu vielen herkömmlichen Anordnungen externer Funktionseinrichtungen können die Anbringung und Abstützung der zweiten Drainage- bzw. Stützstruktur erfindungsgemäß befriedigend gelöst werden. Die zweite Stützstruktur kann in vorteilhafter Weise präzise, ausreichend steif und ausreichend druckkraftübertragend und dabei drainagefähig vor der Filtermembranfläche angebracht werden.

Da bei der zweiten Stützstruktur erfindungsgemäß bewusst auf Präzision und Steifigkeit verzichtet werden kann, können vorteilhaft weiter Kosten eingespart werden.

Im Gegensatz zu Schweißverbindungen zwischen gleichartigen oder beidseitig aufschmelzenden Verbindungspartnern, bei denen sich zwar der gleiche Dichteffekt zwischen den Stützstrukturen und der Filtermembran ergeben würde, aber mit einer geringeren mechanischen Belastbarkeit der Verbindung einhergehen, kann erfindungsgemäß durch Auswählen eines niederschmelzenden Materials für die Fluidzuführungskammer eine dichte und zugleich mechanisch gut belastbare Verbindung erreicht werden. Auf diese Weise kann vorteilhaft vermieden werden, dass die erste Stützschicht durch das Aufschmelzen und Verpressen dünner wird. Ferner können vorteilhaft Materialfehler und/oder ungünstige Querschnittssprünge an den Übergängen zu den nicht aufgeschmolzenen Bereichen der Filtermembran vermieden werden.

Weiterhin ist mit der erfindungsgemäß vorgesehenen zweiten Stützstruktur eine gute Zugänglichkeit der Filtermembran aus seitlicher Richtung, in der Ebene der Filtermembran, möglich. Dies kann insbesondere bei ungewollter Benetzung der Filtermembran mit Flüssigkeit von Vorteil sein.

Durch die höhere Drainagefähigkeit zum Inneren der Fluidaufnahmekammer hin können sich vorteilhaft wichtige Sicherheitsvorteile ergeben: Bei herkömmlichen Filtermembrananordnungen mit zur Flüssigkeitsoberfläche parallelen Filtermembranen kann es bei unzulässiger vollständiger Flutung bis an die Filtermembran zu einem Druckstoß kommen, welcher sowohl zur Filtermembranzerstörung wie auch zur Zerstörung oder Irritation weiterer Komponenten der externen Funktionseinrichtung und/oder der Behandlungsvorrichtung führen kann. Mit der erfindungsgemäßen Einrichtung kann jedoch ein unzulässig weiter steigender Pegel kontinuierlich die Filtermembranfläche überstreichen, so dass sich der Durchflussdruck sanft ansteigend und stoßfrei erhöhen kann.

Daneben kann die erfindungsgemäß vorgesehene Anordnung der Filtereinrichtung vorteilhaft einer mehrfachen gefahrlosen Wiederholbarkeit des Fehlerfalls einer vollständigen Flutung der Fluidaufnahmekammer Stand halten, da die Flüssigkeit in der vorliegenden Erfindung in der Lage ist, unter Schwerkrafteinfluss selbsttätig wieder von der Filtermembran in die Fluidaufnahmekammer abzulaufen.

Ferner kann es vorteilhaft möglich sein, diese Erscheinung sogar durch eine entsprechende Auswertung dazu zu nutzen, einen Fehlerfall zu erkennen und sinnvolle Gegenmaßnahmen einzuleiten.

Auf diese Weise kann es vorteilhaft möglich sein, einen Abbruch des Behandlungsverfahrens aufgrund von Steuerungsfehlern der Behandlungsvorrichtung zu vermeiden.

Die erfindungsgemäße Vorrichtung zur Sterilluftversorgung von Einmalteilanordnungen behebt die Nachteile der aktuellen Systeme auf einfache und dabei kostenreduzierende Weise und bringt darüber hinaus weitere Vorteile für die Systemumgebung aus Behandlungsvorrichtung und externer Funktionseinrichtung mit sich.

Die erfindungsgemäße Anordnung ist vorteilhaft zur Anwendung in allen fluidischen Gesamtanordnungen aus Komponenten der Behandlungsvorrichtung und fluidführenden Komponenten der externen Funktionseinrichtung geeignet, welche über die geschilderten Verpressungseinrichtungen verfügen.

Die Bauart der verwendbaren Anordnungen der externen Funktionseinrichtung ist in vorteilhafter Weise nicht nur auf Kassettenbauarten beschränkt, die aus Hartteilkassetten mit Folienabdeckungen bestehen. Auch Einzelbehälteranordnungen, welche bisher nicht als Kassettenkonstruktionen gedacht werden, können im Sinne der erfindungsgemäßen Einrichtung umgestaltet werden, so dass das wichtige Merkmal sicher funktionierender steriler Hydrophobmembranen wirtschaftlich zu erreichen ist. In einem solchen Fall kann das Abdeckungselement, wie eine Folie, beispielsweise durch nachgiebig gestaltete Spritzgusselemente ersetzt werden.

Durch geeignete Verpressung der erfindungsgemäßen Einrichtung als Teil einer externen Funktionseinrichtung mit einer Behandlungsvorrichtung können ferner vorteilhaft auf einfache und kostengünstige Weise sowohl eine gute Abstützung wie auch eine optimale Ausrichtung der Filtereinrichtung erreicht werden (Geradebiegen der externen Funktionseinrichtung und der Stützstrukturen).

Die erfindungsgemäße Einrichtung kann in vorteilhafter Weise eine eigene Funktionalität des Toleranzausgleichs und der Kraftbegrenzung aufweisen. Hierfür können in vorteilhafter Weise mindestens fünf Möglichkeiten auf einfache Weise parallel zu Verfügung gestellt werden: Ein federnd ausgeführter maschinenseitiger Fluidkonnektor (in der Regel ein Gummistopfen, welcher zur Abdichtung dienen kann), eine wie eine gewölbte Federscheibe nachgiebiger und longitudinal wie kardanisch federnd und an die Behandlungsvorrichtung sich ausrichtend wirksamer flacher Boden der Fluidzuführungskammer (in Verbindung mit dem im Wesentlichen mittig aufgebrachten Fluidkonnektor und winkelnachgiebiger Konnektion zur Behandlungsvorrichtung, eine gegebenenfalls auch in Anpressrichtung federnd nachgiebige Gestaltung der Stützstrukturen (etwa durch Wellenform oder durch Noppen, welche toleranzausgleichend und federnd in eine Gummimatte der Behandlungsvorrichtung eindringen können), durch eine lokal unter der zweiten Stützstruktur gesondert nachgiebig gestaltete Gummimatte (etwa mit Prismen oder Noppen) oder durch eine nachgiebige oder federnde Struktur in der korpusseitigen Struktur der Behandlungsvorrichtung (zum Beispiel Federn oder Noppen).

Im Folgenden wird die vorliegende Erfindung unter Bezugnahme auf die beigefügte Zeichnung beschrieben. In der Zeichnung bezeichnen identische Bezugszeichen gleiche oder identische Elemente. Es zeigt:
- Fig. 1: eine Draufsicht auf die erfindungsgemäße Einrichtung;
- Fig. 2: einen Querschnitt durch eine erfindungsgemäße Einrichtung; und
- Fig. 3: einen Ausschnitt der erfindungsgemäßen Einrichtung aus Fig. 2 in Vergrößerung im Längsschnitt.

**Fig. 1** zeigt eine Draufsicht auf eine Einrichtung gemäß einer Ausführungsform der vorliegenden Erfindung.

Die Einrichtung 100 weist eine Fluidaufnahmekammer 1 sowie eine Fluidzuführungskammer 3 auf.

In der Fluidaufnahmekammer 1 befindet sich ein erstes Fluid 5, welches bis zu einem maximalen Fluidspiegel 7 in die Fluidaufnahmekammer 1 eingefüllt ist.

Die Fluidzuführungskammer 3 weist eine Filtereinrichtung auf, von welcher in Fig. 1 eine Filtermembran 9 sowie eine erste Stützstruktur 11 gezeigt sind. Die erste Stützstruktur 11 weist eine Drainagestruktur 13 auf. Die erste Stützstruktur 11 ist über Befestigungseinrichtungen 15, beispielsweise Nietverbindungen, mit der Fluidzuführungskammer 3 verbunden.

Ein Fluidkonnektor 17 ist mit der ersten Stützstruktur 11 der Filtereinrichtung verbunden.

Durch den Fluidkonnektor 17 wird ein zweites Fluid 19 in die Fluidaufnahmekammer 1 eingebracht. Das zweite Fluid 19 kann zum Behandeln des ersten Fluids 5, beispielsweise durch Aufbringen eines Drucks, verwendet werden.

Wie in der vorliegenden Fig. 1 gezeigt, stellt die Fluidaufnahmekammer 1 einen unteren Bereich 21 und die Fluidzuführungskammer 3 einen oberen Bereich 23 dar.

**Fig. 2** zeigt eine erfindungsgemäße Einrichtung im Querschnitt. Die Einrichtung 100 ist hier als Teil einer externen Funktionseinrichtung 200 dargestellt, welche mit einer Behandlungsvorrichtung 300 verpresst ist.

Der untere Bereich 21 der Einrichtung 100 bildet den tieferen Bereich, also die Fluidaufnahmekammer 1. Der obere Bereich 23 bildet den flacheren Bereich, also die Fluidzuführungskammer 3.

Wie in Fig. 2 mit dem Doppelpfeil gezeigt ist, wird ein zweites Fluid 19 durch einen Fluidkonnektor 17 in die Fluidzuführungskammer 3 eingebracht.

Die Fluidzuführungskammer 3 weist, wie in Fig. 2 gezeigt ist, eine erste Stützstruktur 11 auf, welche mittels einer dichten Verbindung 25, beispielsweise einer Schweiß- oder Klebverbindung, an der Fluidzuführungskammer 3 befestigt ist.

Auf der ersten Stützstruktur 11 ist die Filtermembran 9 angeordnet, auf welcher wiederum die zweite Stützstruktur 27 vorgesehen ist.

Die externe Funktionseinrichtung 200 ist an einer Seite mit einem Abdeckungselement 29, beispielsweise einer Folie, abgedeckt.

Über eine Gummimatte 31, welche zum Übertragen von Kraft und/oder Bewegungen durch Sensoren oder Aktoren der Behandlungsvorrichtung 300 auf die externe Funktionseinrichtung 200 bzw. Kammern und/oder Kanäle derselben dienen kann, wird die externe Funktionseinrichtung 200 mit der Behandlungsvorrichtung 300 verpresst.

Die Gummimatte 31 kann eine gezielte Nachgiebigkeitsstruktur 33 aufweisen.

Zum Einbringen oder Entfernen der im Inneren der Fluidaufnahmekammer 1 befindlichen Fluide kann die Fluidaufnahmekammer 1 an ihrer Unterseite mit einem Fluidanschluss 35 versehen sein.

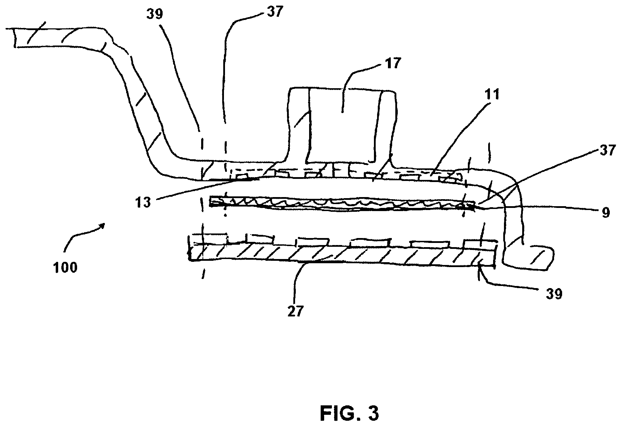

**Fig. 3** zeigt einen vergrößerten Ausschnitt bzw. Abschnitt der erfindungsgemäßen Einrichtung 100 aus Fig. 2, genauer gesagt, die Filtereinrichtung im Längsschnitt.

Im Vergleich zur Darstellung der Fig. 2 wurde die Filtereinrichtung um 90° gekippt, so dass der Fluidkonnektor 17 für das zweite Fluid in der Darstellung der Fig. 3 nach oben gerichtet ist.

Die Filtermembran 9, welche exemplarisch eine Membranschicht und eine (nicht dargestellte) dritte Stützschicht in Form eines Gewebes aufweist, ist zwischen der ersten Stützstruktur 11 und der zweiten Stützstruktur 27 angeordnet.

Zur detaillierten Beschreibung der einzelnen Komponenten wird auf die obigen Ausführungen verwiesen.

Zum Herstellen der dichten Verbindung zwischen der ersten Stützstruktur 11 und der Filtermembran 9, ist eine erste Ringzone bzw. ein erster Ringbereich 37 vorgesehen. Zum Herstellen der Verbindung zwischen der Filtermembran 9 und der zweiten Stützstruktur 27 ist eine zweite Ringzone bzw. ein zweiter Ringbereich 39 vorgesehen.

## Patentansprüche

1. Externe Funktionseinrichtung (200) mit einer Einrichtung (100), wobei die Einrichtung (100) aufweist:
- wenigstens eine Fluidaufnahmekammer (1) zum Aufnehmen wenigstens eines ersten medizinischen Fluids (5); und
- wenigstens eine hydrophobe Filtereinrichtung mit einer Filteroberfläche,
**dadurch gekennzeichnet,**
- **dass** die Einrichtung (100) wenigstens eine Fluidzuführungskammer (3) aufweist zum Zuführen eines zweiten Fluids, wobei die Fluidzuführungskammer (3) in wenigstens einem Abschnitt hiervon mit der Fluidaufnahmekammer (1) verbunden ist;
- **dass** die Fluidzuführungskammer (3) wenigstens einen Fluidkonnnektor (17) aufweist, durch welchen der Fluidzuführungskammer (3) ein zweites gasförmiges Fluid (19), insbesondere Luft, zuführbar ist;
- **dass** der Fluidaufnahmekammer (1) durch die Filtereinrichtung das zweite Fluid (19) zuführbar ist;
- **dass** die Fluidzuführungskammer (3) und die Fluidaufnahmekammer (1) lediglich über die Filtereinrichtung miteinander in Fluidkommunikation stehen; und
- **dass** ein Normalenvektor auf die Filteroberfläche der hydrophoben Filtereinrichtung nicht parallel zu einem Normalenvektor auf einen Fluidspiegel (7) des im Gebrauch in der Fluidaufnahmekammer (1) aufgenommenen ersten Fluids (5) verläuft; und
- **dass** die Filtereinrichtung wenigstens eine Filtermembran (9) aufweist und wobei die Filtereinrichtung auf wenigstens einer Seite der Filtermembran (9) eine Stützstruktur aufweist.

2. Externe Funktionseinrichtung (200) nach Anspruch 1, wobei der Normalenvektor auf die Filteroberfläche der hydrophoben Filtereinrichtung im Wesentlichen senkrecht zum Normalenvektor auf die Ebene des Fluidspiegels (7) ist.

3. Externe Funktionseinrichtung (200) nach Anspruch 1 oder 2, wobei der Normalenvektor auf die Filteroberfläche keinen Schnittpunkt mit einem Fluidspiegel (7) des in der Fluidaufnahmekammer (1) vorhandenen ersten Fluids (5) aufweist.

4. Externe Funktionseinrichtung (200) nach Anspruch 1, wobei die Filtereinrichtung in einem Inneren der Fluidzuführungskammer (3) angeordnet ist.

5. Externe Funktionseinrichtung (200) nach Anspruch 1 wobei die Stützstruktur eine erste Stützstruktur (11) aufweist, welche auf der dem Inneren der Fluidzuführungskammer (3) zugewandten Seite der Filtermembran (9) angeordnet ist.

6. Externe Funktionseinrichtung (200) nach Anspruch 5, wobei die Stützstruktur eine erste Stützstruktur (11) aufweist, welche in wenigstens einem äußeren Bereich derselben stoffschlüssig oder zusätzlich durch Verschweißen mit einem Verbindungsbereich der Fluidzuführungskammer (3) verbunden ist.

7. Externe Funktionseinrichtung (200) nach Anspruch 5 oder 6, wobei die erste Stützstruktur (11) wenigstens in einem äußeren Bereich derselben aus einem höher schmelzenden Werkstoff als ein Verbindungsbereich der Fluidzuführungskammer (3) hergestellt ist.

8. Externe Funktionseinrichtung (200) nach einem der Ansprüche 5 bis 7, wobei eine zweite Stützstruktur (27) auf der dem Inneren der Fluidzuführungskammer (3) abgewandten Seite der Filtermembran (9) angeordnet ist.

9. Externe Funktionseinrichtung (200) nach Anspruch 8, wobei die zweite Stützstruktur (27) die Filtermembran (9) im Wesentlichen vollständig überdeckt.

10. Externe Funktionseinrichtung (200) nach einem der vorangegangenen Ansprüche, wobei die Einrichtung (100) stoffschlüssig in eine externe Funktionseinrichtung (200) integriert ist.

11. Externe Funktionseinrichtung (200) gemäß einem der Ansprüche 1 bis 10 zum Behandeln wenigstens eines Fluids, welche als Blutkassette ausgestaltet ist.

12. Externe Funktionseinrichtung nach Anspruch 11, wobei die Filtereinrichtung der Einrichtung (200) parallel zu einem Abdeckungselement (29) der Einrichtung (100) zum Verschließen eines Inneren der Fluidaufnahmekammer (1) gegenüber einem Äußeren angeordnet ist, wobei das Abdeckungselement (29) als Abdeckfolie ausgestaltet ist.

13. Externe Funktionseinrichtung (200) nach einem der vorangegangenen Ansprüche, wobei das erste medizinische Fluid (5) Blut ist oder umfasst.

14. Behandlungsvorrichtung zum Behandeln wenigstens eines Fluids, welche eine externe Funktionseinrichtung (200) gemäß einem der Ansprüche 1 bis 13 aufweist, wobei die Behandlungsvorrichtung eine Blutbehandlungsvorrichtung ist.

## Claims

1. An external functional device (200) with a device (100), wherein the device (100) comprises:
- at least one fluid receiving chamber (1) for receiving at least one first medical fluid (5); and
- at least one hydrophobic filter device with a filter surface,
**characterized in that**,
- the device (100) comprises at least one fluid supply chamber (3) for supplying a second fluid, wherein the fluid supply chamber (3) is connected to the fluid receiving chamber (1) in at least a portion thereof;
- the fluid supply chamber (3) comprises at least one fluid connector (17) through which a second gaseous fluid (19), in particular air, can be supplied to the fluid supply chamber (3);
- the second fluid (19) can be supplied to the fluid receiving chamber (1) through the filter device;
- the fluid supply chamber (3) and the fluid receiving chamber (1) are only in fluid communication with each other via the filter device; and
- a normal vector on the filter surface of the hydrophobic filter device is not parallel to a normal vector on a fluid level (7) of the first fluid (5) received in the fluid receiving chamber (1) in use; and
- the filter device comprises at least one filter membrane (9) and wherein the filter device comprises one supporting structure on at least one side of the filter membrane (9) .

2. The external functional device (200) according to claim 1, wherein the normal vector on the filter surface of the hydrophobic filter device is substantially perpendicular to the normal vector on the plane of the fluid level (7).

3. The external functional device (200) according to claim 1 or 2, wherein the normal vector on the filter surface doesn't comprise any point of intersection with a fluid level (7) of the first fluid (5) present in the fluid receiving chamber (1).

4. The external functional device (200) according to claim 1, wherein the filter device is arranged in an interior of the fluid supply chamber (3).

5. The external functional device (200) according to claim 1, wherein the support structure comprises a first support structure (11) arranged on the side of the filter membrane (9) facing the interior of the fluid supply chamber (3).

6. The external functional device (200) according to claim 5, wherein the support structure comprises a first support structure (11), which is material-locked or additionally connected by welding to a connection region of the fluid supply chamber (3) in at least one outer region thereof.

7. The external functional device (200) according to claim 5 or 6, wherein the first support structure (11) is made, at least in an outer area thereof, of a higher melting material than a connection region of the fluid supply chamber (3).

8. The external functional device (200) according to anyone of claims 5 to 7, wherein a second support structure (27) is arranged on the side of the filter membrane (9) opposite to the interior of the fluid supply chamber (3).

9. The external functional device (200) according to claim 8, wherein the second support structure (27) substantially completely covers the filter membrane (9).

10. The external functional device (200) according to anyone of the preceding claims, wherein the device (100) is material-locked integrated into an external functional device (200).

11. The external functional device (200) according to anyone of the claims 1 to 10 for treating at least one fluid, which is configured as a blood cassette.

12. The external functional device (200) according to claim 11, wherein the filter device of the device (200) is arranged parallel to a cover element (29) of the device (100) for closing an interior of the fluid receiving chamber (1) from an exterior, the cover element (29) being configured as a cover foil.

13. The external functional device (200) according to anyone of the preceding claims, wherein the first medical fluid (5) is or contains blood.

14. A treatment apparatus for treating at least one fluid, which comprises an external functional device (200) according to anyone of claims 1 to 13, wherein the treatment apparatus is a blood treatment apparatus.

## Revendications

1. Un dispositif fonctionnel externe (200) doté d'un dispositif (100), où le dispositif (100) comprend:
- au moins une chambre de réception de fluide (1) pour recevoir au moins un premier fluide médical (5); et
- au moins un dispositif de filtration hydrophobe doté d'une surface filtrante,
**caractérisé en ce que**,
- le dispositif (100) comprend au moins une chambre d'alimentation en fluide (3) pour amener un second fluide, où la chambre d'alimentation en fluide (3) est reliée à la chambre de réception de fluide (1) dans au moins une section de celle-ci;
- la chambre d'alimentation en fluide (3) comprend au moins un connecteur fluidique (17) au travers duquel un second fluide gazeux (19), en particulier de l'air, peut être amené à la chambre d'alimentation en fluide (3);
- le second fluide (19) peut être amené à la chambre de réception de fluide (1) par le dispositif de filtration;
- la chambre d'alimentation en fluide (3) et la chambre de réception de fluide (1) sont en communication fluidique l'une avec l'autre uniquement par l'intermédiaire du dispositif de filtration; et
- un vecteur normal à la surface filtrante du dispositif de filtration hydrophobe n'est pas parallèle à un vecteur normal à un niveau fluidique (7) du premier fluide (5) reçu dans la chambre de réception de fluide (1) en cours d'utilisation; et
- le dispositif de filtration comprend au moins une membrane filtrante (9) et où le dispositif de filtration comprend une structure de support sur au moins un côté de la membrane filtrante (9).

2. Le dispositif fonctionnel externe (200) selon la première revendication, où le vecteur normal à la surface de filtration hydrophobe est pratiquement perpendiculaire au vecteur normal au plan du niveau fluidique (7).

3. Le dispositif fonctionnel externe (200) selon la revendication 1 ou 2, où le vecteur normal à la surface de filtration ne comprend aucun point d'intersection avec un niveau fluidique (7) du premier fluide (5) présent dans la chambre de réception de fluide (1).

4. Le dispositif fonctionnel externe (200) selon la première revendication, où le dispositif de filtration est agencé à l'intérieur de la chambre d'alimentation en fluide (3).

5. Le dispositif fonctionnel externe (200) selon la première revendication, où la structure de support comprend une première structure de support (11) agencée sur le côté de la membrane filtrante (9) faisant face l'intérieur de la chambre d'alimentation en fluide (3).

6. Le dispositif fonctionnel externe (200) selon la revendication 5, où la structure de support comprend une première structure de support (11) reliée de manière adhérente ou par soudage additionnel à une zone de liaison de la chambre d'alimentation en fluide (3) dans au moins une zone extérieure de celle-ci.

7. Le dispositif fonctionnel externe (200) selon la revendication 5 ou 6, où la première structure de support (11) est constituée, au moins dans une zone extérieure de celle-ci, d'un matériau dont le point de fusion est plus élevé qu'une zone de liaison de la chambre d'alimentation en fluide (3).

8. Le dispositif fonctionnel externe (200) selon l'une quelconque des revendications 5 à 7, où une seconde structure de support (27) est agencée sur le côté de la membrane filtrante (9) opposé à l'intérieur de la chambre d'alimentation en fluide (3).

9. Le dispositif fonctionnel externe (200) selon la revendication 8, où la seconde structure de support (27) recouvre pratiquement complètement la membrane filtrante (9).

10. Le dispositif fonctionnel externe (200) selon l'une quelconque des revendications précédentes, où le dispositif (100) est intégré de manière adhérente dans un dispositif fonctionnel externe (200).

11. Le dispositif fonctionnel externe (200) selon l'une quelconque des revendications 1 à 10 pour traiter au moins un fluide, qui est conçu comme une cassette à sang.

12. Le dispositif fonctionnel externe (200) selon la revendication 11, où le dispositif de filtration du dispositif (200) est agencé parallèlement à un élément de recouvrement (29) du dispositif (100) de façon à fermer un intérieur de la chambre de réception de fluide (1) par rapport à l'extérieur, l'élément de recouvrement (29) étant conçu sous la forme d'un film de recouvrement.

13. Le dispositif fonctionnel externe (200) selon l'une quelconque des revendications précédentes, où le premier fluide médical (5) est ou contient du sang.

14. Un appareil de traitement pour traiter au moins un fluide, qui comprend un dispositif fonctionnel externe (200) selon l'une quelconque des revendications 1 à 13, où l'appareil de traitement est un appareil de traitement du sang.
